# EUROPEAN PATENT SPECIFICATION

(11) **EP 3 094 361 B1**
(45) Date of publication and mention of the grant of the patent: **14.03.2018**
(21) Application number: 15700980.4
(22) Date of filing: 12.01.2015
(51) Int. Cl.: A61M 5/145, A61M 5/20

(54) **TRANSMISSION ARRANGEMENT FOR MOTORIZED DRUG DELIVERY DEVICE**
GETRIEBEANORDNUNG FÜR MOTORISIERTE ARZNEIMITTELABGABEVORRICHTUNG
AGENCEMENT DE TRANSMISSION POUR DISPOSITIF D'ADMINISTRATION DE MÉDICAMENT MOTORISÉ

(30) Priority: 13.01.2014 EP 14150909
(43) Date of publication of application: 23.11.2016
(73) Proprietor: Novo Nordisk A/S, 2880 Bagsværd (DK)
(72) Inventor: HØHOLT, Jesper, DK-2880 Bagsværd (DK); ANDERSEN, Carsten, Schau, DK-2880 Bagsværd (DK); LARSEN, Bjørn, Gullak, DK-2880 Bagsværd (DK); SØRENSEN, Thomas, DK-2880 Bagsværd (DK); BROCKMEIER, Pete, DK-2880 Bagsværd (DK); MEWS, Steffen, DK-2880 Bagsværd (DK)
(86) International application number: PCT/EP2015/050417
(87) International publication number: WO 2015/104412

(56) References cited:
- GB-A- 2 094 904
- US-A1- 2012 191 043

## Description

The present invention generally relates to a transmission arrangement. In a specific aspect, the invention relates to a motorized drug delivery device adapted to receive a drug filled cartridge and subsequently expel a dose therefrom.

### BACKGROUND OF THE INVENTION

In the disclosure of the present invention reference is mostly made to the treatment of diabetes by subcutaneous drug delivery, either discrete or continuous, however, this is only an exemplary use of the present invention.

The most common type of durable drug delivery devices adapted to receive a drug filled cartridge and expel a discrete dose of a desired size therefrom are driven by manual means or by a spring energized during dose setting, the cartridge being of the type comprising an axially displaceable piston having an initial proximal position and which is moved distally by a piston rod. Subcutaneous drug delivery takes place via an injection needle arranged in fluid communication with the cartridge. The device may be pen-formed or in the form of a more box-shaped so-called doser. In order to improve convenience, user-friendliness and provide additional features, e.g. detection and storing of expelling data, drug delivery devices have been provided with electrically driven means, typically in the form of an electronically controlled motor driving a piston rod through a gear arrangement, e.g. as shown in US 6,514,230 and US 2011/306927.

Whereas motorized drug delivery devices for treatment of diabetes by discrete injections of e.g. insulin are used relatively rarely, in the field of continuous drug delivery motorized drug delivery devises have been used widely for decades. The latter type of devices are generally known as infusion pumps and are normally engineered to very high standards and are correspondingly very expensive. Although a motorized drug delivery device for discrete injections of drug also has to meet very high safety standards, the cost issue is more important as the relatively inexpensive mechanical drug delivery devices, e.g. of the pen-type, to most users are an acceptable alternative.

Motorized drug delivery devices, either of the pen or pump type, often have a two-axis design in which rotation is transferred form a motor arranged corresponding to a first to an axially displaceable drive member, e.g. a piston rod, arranged corresponding to a second axis. In most cases transfer between the two axes takes place using a transmission with gear wheels. To secure reliable and durable transmission in a compact design high precision on the components is required to avoid misalignment in the transmission. Examples of two-axis designs are known from e.g. US 2012/0191043, forming the basis for the two-part form of claim 1, and US 6,854,620 disclosing infusion pumps and US 2012/179112 disclosing a doser-type injection device, all including a drive mechanism comprising a motor and a transmission gear arrangement capable of linearly displacing a piston rod.

Having regard to the above, it is an object of the present invention to provide a motorized drug delivery device as well as components therefor which provide a high degree of reliability in a cost-effective way.

### DISCLOSURE OF THE INVENTION

In the disclosure of the present invention, embodiments and aspects will be described which will address one or more of the above objects or which will address objects apparent from the below disclosure as well as from the description of exemplary embodiments.

Thus, in accordance with a first aspect of the invention a transmission is provided comprising a first axis and a second axis which in a reference position are arranged in parallel with each other, the transmission comprising first and second gear wheels and a rod. The first gear wheel has a first centre plane, and an axis of rotation corresponding to the first axis. The second gear wheel has a second centre plane, a reference axis of rotation corresponding to the second axis, and a threaded bore corresponding to the second axis. The rod is arranged non-rotationally relative to the second gear wheel and has an outer thread along at least a part of its length, the rod in a reference position being arranged corresponding to the second axis and in threaded engagement with the threaded bore, rotation of the second gear wheel thereby providing axial movement of the rod. The first and second gear wheel, when in the reference position, are arranged with the first and second centre plane in a common plane and in rotational engagement with each other, wherein the combined second gear wheel and rod are arranged to pivot corresponding to a centre point defined by the intersection of the second axis and the second centre plane, whereby the rod, with the gear wheels in rotational engagement, can be arranged out of alignment with the first axis.

By such an arrangement a transmission is provided which provides a high degree of reliability in a cost-effective way. More specifically, the above-described pivoting between the different components can be used to compensate for "imperfections" of the transmission: (i) static misalignment during assembly due to "loose" tolerances of the different components are accommodated, (ii) dynamic misalignment during operation can be accommodated, e.g. the piston rod may wobble if not perfectly straight, and the gear wheels may twist relative to the rod during load.

In the context of the present disclosure the term "pivot" is generally used to describe "smaller" rotational movements, e.g. less than 10 degrees, bringing the components out of a reference position and having an axis different from the reference axis of rotation.

In an exemplary embodiment the transmission comprises a ball joint assembly having a ball portion comprising the second gear wheel, and a ball housing, whereby the ball joint allows the combined second gearwheel and rod to pivot corresponding to the centre point.

The ball portion may comprise a gear wheel member comprising the threaded bore and a first circumferential ball bearing surface arranged perpendicularly relative to the second axis, a ball housing comprising a second circumferential ball bearing surface arranged perpendicularly relative to the second axis, and a rotational bearing member arranged between the gear wheel member and the ball housing and perpendicularly relative to the second axis. In such an arrangement the first and second circumferential ball bearing surfaces in combination with corresponding bearing surfaces on the ball housing form first and second circumferential bearings which in combination provides the ball joint. The friction of the rotational bearing is lower than the rotational friction of the second circumferential bearing. The radius of the first circumferential bearing may be smaller than the radius of the second circumferential bearing.

The above-described transmission may be incorporated in a drug delivery device which further comprises a compartment adapted to receive a drug-filled cartridge, the cartridge comprising a body portion, an axially displaceable piston, and a distal outlet portion adapted to be arranged in fluid communication with a flow conduit, wherein the rod is adapted to directly or indirectly engage and axially move the piston of a loaded cartridge to thereby expel drug from the cartridge, the drug delivery device further comprising an electronically controlled drive arrangement adapted to rotate the first gear wheel. The drive arrangement may comprise a motor and a controller for controlling a motor. The controller may be associated with or comprise a receiver and/or transmitter allowing the device to communicate with an external source, e.g. by wireless means with a smartphone. In this way a log of expelled doses could be transferred to a smartphone or the smartphone could be used to conveniently enter pre-set dose sizes.

The drug delivery device may be provided with setting means allowing a user to set a dose of drug to be expelled. The setting means may be in the form of a setting device, e.g. one or more user input keys, or the above-mentioned wired or wireless receiver adapted to receive setting input from an external source such as a PC or a smartphone.

As used herein, the term "drug" is meant to encompass any flowable medicine formulation capable of being passed through a delivery means such as a cannula or hollow needle in a controlled manner, such as a liquid, solution, gel or fine suspension, and containing one or more drug agents. Representative drugs include pharmaceuticals such as peptides (e.g. insulins, insulin containing drugs, GLP-1 containing drugs as well as derivates thereof), proteins, and hormones, biologically derived or active agents, hormonal and gene based agents, nutritional formulas and other substances in both solid (dispensed) or liquid form. In the description of the exemplary embodiments reference will be made to the use of insulin containing drugs, this including analogues thereof as well as combinations with one or more other drugs.

### BRIEF DESCRIPTION OF THE DRAWINGS

In the following exemplary embodiments of the invention will be further described with reference to the drawings, wherein
fig. 1 shows in a cross-sectional view a drug delivery device with a transmission assembly,
fig. 2 shows an exploded view of the transmission assembly of fig. 1, and
fig. 3 shows a cross-sectional view of the transmission assembly of fig. 1.

In the figures like structures are mainly identified by like reference numerals.

### DESCRIPTION OF EXEMPLARY EMBODIMENTS

When in the following terms such as "upper" and "lower", "right" and "left", "horizontal" and "vertical" or similar relative expressions are used, these only refer to the appended figures and not necessarily to an actual situation of use. The shown figures are schematic representations for which reason the configuration of the different structures as well as their relative dimensions are intended to serve illustrative purposes only. When the term member or element is used for a given component it generally indicates that in the described embodiment the component is a unitary component, however, the same member or element may alternatively comprise a number of sub-components just as two or more of the described components could be provided as unitary components, e.g. manufactured as a single injection moulded part. The term "assembly" does not imply that the described components necessarily can be assembled to provide a unitary or functional assembly during a given assembly procedure but is merely used to describe components grouped together as being functionally more closely related.

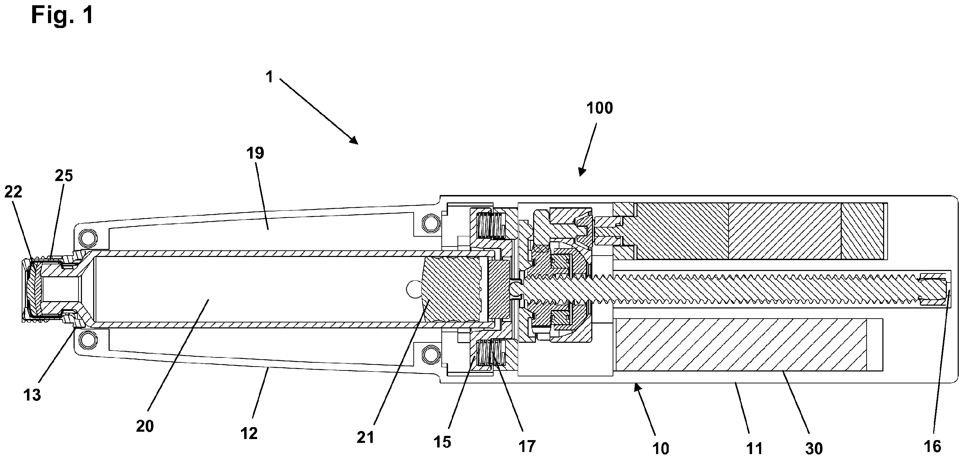

Referring to fig. 1 a generally pen-formed drug delivery device 1 will be described. More specifically, the pen device comprises a cap part (not shown) and a main part 10 having a proximal body or drive assembly portion with a housing 11 in which a motorized drug expelling assembly 100 (described in greater detail below) and an electronic controller 30 with an electric power source are arranged, and a distal cartridge holder portion 12 with a compartment 19 in which a drug-filled cartridge 20 is arranged and retained in place. The power source may be rechargeable with the device further comprising an electrical connector allowing an external connector of a power source to be connected for recharging the power source. The controller may be associated with or comprise a receiver and/or transmitter allowing the device to communicate with an external source, e.g. by wireless means with a smartphone. In this way a log of expelled doses could be transferred to a smartphone or the smartphone could be used to conveniently enter pre-set dose sizes.

The cartridge comprises a generally cylindrical main portion with an axially displaceable piston 21 and a distal outlet portion comprising a needle-penetrable septum 22. The cartridge is further provided with distal coupling means in the form of a needle hub mount 25 having, in the shown example, an external thread adapted to engage an inner thread of a corresponding hub of a needle assembly. The cartridge may for example contain an insulin, a GLP-1 or a growth hormone formulation. The device further comprises dose setting means allowing a user to set a dose of drug to be expelled as well as a display showing the set dose (not shown). The display may be adapted to show text or the display may be a simple 7-segment display which could be adapted to display one or more additional symbols.

In the shown embodiment the device is designed to be loaded by the user with a new cartridge through a distal receiving opening 13 in the cartridge holder assembly, the cartridge holder comprising closure means (not shown) operatable by a user between an open position in which a cartridge can be inserted respectively removed, and a closed position in which an inserted cartridge is held in place. In order to axially position the cartridge, the device comprises a seat member 15 adapted to receive the proximal end of the cartridge, the seat member being biased in the proximal direction by springs 17 thereby forcing the cartridge into contact with the closure means.

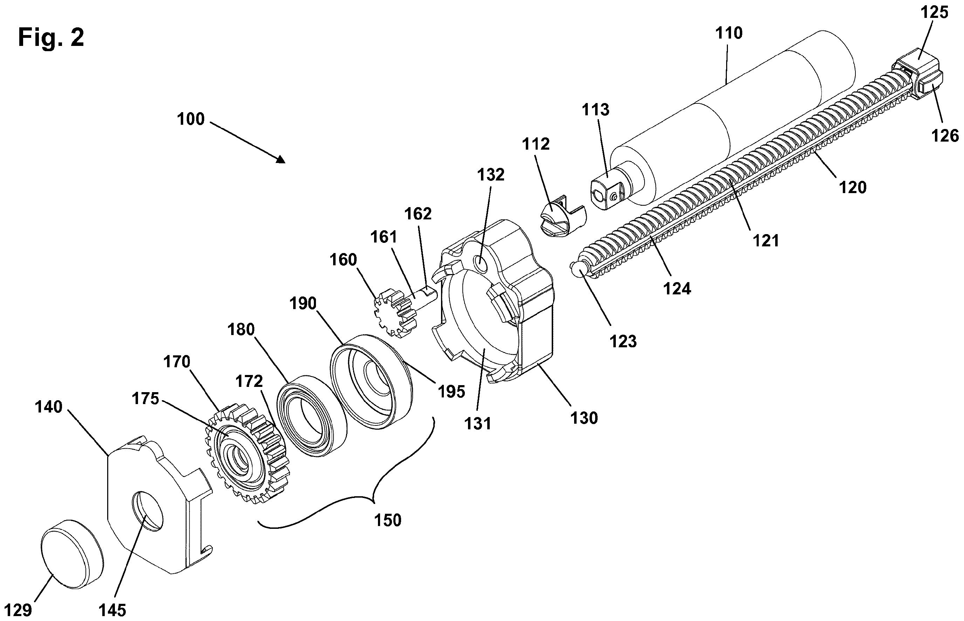

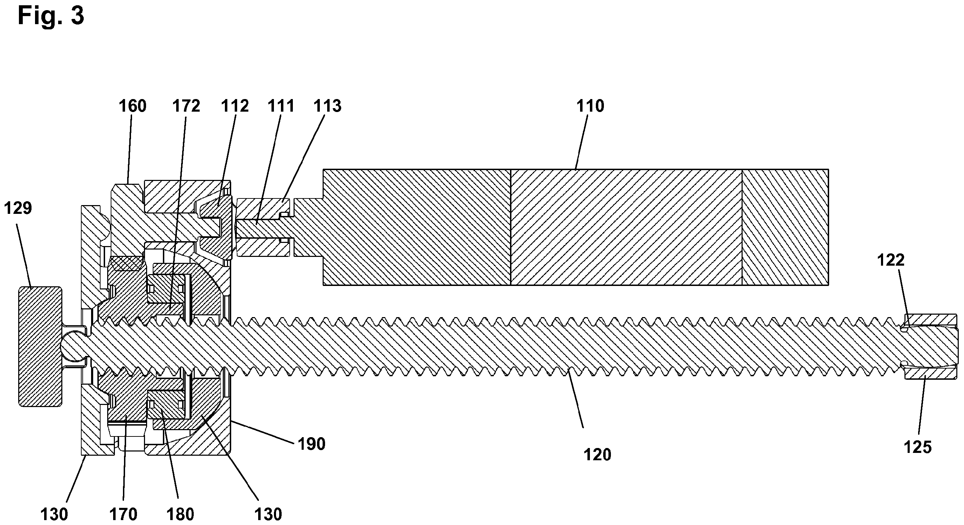

Turning to fig. 2 the motorized drug expelling assembly 100 will be described. The assembly comprises a motor assembly adapted to be mounted within the housing, a gear box assembly adapted to be mounted within the housing, and a piston rod assembly adapted to be mounted axially displaceable in the housing. The rotational axes for the different rotating components are arranged generally co-axially in the Z-direction as defined e.g. by the longitudinal axis of the piston rod. Referring to figs. 1 and 3, the Z- and X-axes are arranged in the plane of the drawing and the Y-axis perpendicularly thereto. The motor assembly comprises a motor unit 110 with a rotating output shaft 111 (see fig. 1) and a coupling for interfacing with an input shaft of the gear box. In the shown embodiment the coupling is in the form of an Oldham coupling comprising a first member 112 adapted to be mounted on the output shaft 111 (as shown in fig. 3) and a second member 113 adapted to engage a gear box input shaft, the two members being connected by a hinge when assembled. Alternatively a cardan joint or another type of flexible connection could be used. In the shown embodiment the motor unit comprises schematically shown a motor, a gear box and a motor controller.

The piston rod assembly comprises a threaded piston rod 120, a guide member 125 and a piston washer 129. The piston rod comprises an external thread 121, a proximal coupling portion 122 (see fig. 3) adapted to engage the guide member, and a distal ball-formed coupling head 123 adapted to engage the piston washer. The guide member comprises a through-going opening adapted to engage the piston rod proximal end non-rotationally in respect of the Z-axis yet provide a ball-like joint allowing a small degree of pivoting in respect of the X- and Y-axes. The guide member further comprises a pair of opposed outwardly oriented protrusions 126 adapted to slidingly engage corresponding guide grooves 16 (see fig. 1) associated with the housing, the guide member thereby preventing rotation of the piston rod relative to the housing. As can be seen the outer protrusions have slightly curved faces allowing a small degree of pivoting of the guide member relative to the housing in respect of the X-axis. In the shown embodiment the piston rod comprises a longitudinal groove 124 which is intended to accommodate a wire connection between sensor means (not shown) arranged in the piston washer and the device electronic controller.

The gear box assembly comprises a gear box housing 130, a gear box cover 140, a nut assembly 150, and an input spur gear wheel 160 having a first gear wheel centre plane. The nut assembly comprises an output spur gear wheel 170 having a second gear wheel centre plane, a ball bearing 180 and a bearing housing 190. The gear box housing and the gear box cover are adapted to be snap-fitted to each other to thereby form a ball housing holding the nut assembly and input gear wheel in position. The output spur wheel comprises a central bore with an internal thread 171 adapted to engage the piston rod external thread (the output gear wheel thereby providing what is traditionally termed a "nut member" for the piston rod), as well as a proximally extending portion 172 with an external cylindrical surface adapted to be mounted non-rotationally in the ball bearing inner housing, the ball bearing outer housing being mounted non-rotationally in the bearing housing, the ball bearing thereby providing a low-friction bearing between the output gear wheel and the bearing housing. The distally facing surface of the "nut gear wheel" comprises a circumferential first inner ball joint surface 175 adapted to engage a corresponding circumferential first outer ball joint surface 145 of the gear box cover, and the proximally facing surface of the bearing housing comprises a circumferential second inner ball joint surface 195 adapted to engage a corresponding circumferential second outer ball joint surface 135 of the gear box housing. In the shown embodiment the first ball joint surfaces have a small radius and a narrow width whereas the second ball joint surfaces have a larger radius and a broader with, however, as they have a common centre of rotation arranged in the second gearwheel centre plane corresponding to the nut gearwheel axis, a combined ball joint is formed. As appears, the rotating nut gear wheel is provided with a proximal low-friction bearing in the form of the ball bearing 180 and a distal bearing formed directly between the nut gear wheel and the gear box cover which can be expected to have a somewhat higher friction. In principle the nut assembly could be formed as a single member with the ball joint providing a rotational bearing between both sides of the nut assembly and the gear box housing, however, for polymeric components this would result in rather high friction from the proximal large-diameter bearing. This said, for the intended small pivoting movements between the nut assembly and the gear box housing (see below) the relatively high friction is not essential. The input spur wheel 160 comprises an input shaft 161 rotationally received in gear wheel bore 132 of the gear box housing, the input shaft having a pair of opposed coupling surfaces adapted to non-rotationally engage the coupling member 112. As described above, in the shown embodiment the coupling is in the form of an Oldham coupling providing a non-rotational coupling between the motor output shaft and the gear box input shaft, yet allows relative axial and pivoting movements in other directions. In the mounted state the first and second gear wheel centre planes are arranged in the same plane, with the teeth in rotational force-transmitting engagement.

Turning to fig. 3 the expelling assembly 100 is shown in an assembled state with the gear wheels being arranged in a common plane and engaging each other. Shown in dark hatching the nut assembly "ball" can be identified in the gear box housing. As the two gear wheels are arranged with a slight amount of play, the ball in the ball joint is allowed to pivot a small amount in respect of both the X- and Y-axes without any substantial influence on transmission efficiency. Especially, as the centre of rotation for the nut assembly is the same as the centre for the output gear wheel, the axis distance and thus gear ratio is kept essentially constant. Further, the Oldham coupling between the motor and gear box allows both translation and rotation to take place between the two assemblies, just as the above-described piston rod guide member allows the piston rod proximal end to pivot slightly.13

The above-described allowed movements between the different components can be used to compensate for different "imperfections" of the entire expelling assembly: (i) static misalignment during assembly due to "loose" tolerances of the different components are accommodated, (ii) dynamic misalignment during operation can be accommodated, e.g. the piston rod may wobble if not perfectly straight, and the motor and gear box may twist relative to each other during load.

In the above description of exemplary embodiments, the different structures and means providing the described functionality for the different components have been described to a degree to which the concept of the present invention will be apparent to the skilled reader. The detailed construction and specification for the different components are considered the object of a normal design procedure performed by the skilled person along the lines set out in the present specification.

## Claims

1. A transmission (100) for a motorized drug delivery device, the transmission comprising:
- a first axis and a second axis which in a reference position are arranged in parallel with each other,
- a first gear wheel (160) having:
- a first centre plane, and
- an axis of rotation corresponding to the first axis,
- a second gear wheel (170) having:
- a second centre plane, and
- a reference axis of rotation corresponding to the second axis, **characterized in that** the second gear wheel (170) has a threaded bore (171) corresponding to the second axis, the transmission further comprising:
- a non-rotationally arranged rod (120) having an outer thread (121) along at least a part of its length, the rod in a reference position being arranged corresponding to the second axis and in threaded engagement with the threaded bore, rotation of the second gear wheel thereby providing axial movement of the rod,
wherein:
- the first gear wheel and the second gear wheel in a reference position are arranged with the first centre plane and the second centre plane in a common plane and in rotational engagement with each other,
- the combined second gear wheel and rod are arranged to pivot corresponding to a centre point defined by the intersection of the second axis and the second centre plane, whereby the rod, with the gear wheels in rotational engagement, can be arranged out of alignment with the first axis.

2. A transmission as in claim 1, comprising a ball joint assembly having:
- a ball portion (170, 180, 190) comprising the second gear wheel, and
- a ball housing (130, 140),
whereby the ball joint allows the combined second gear wheel and rod to pivot corresponding to the centre point.

3. A transmission as in claim 2, wherein the ball portion comprises:
- a gear wheel member (170) comprising the threaded bore (171) and a first circumferential ball bearing surface (175) arranged perpendicularly relative to the second axis,
- a bearing housing (190) comprising a second circumferential ball bearing surface (195) arranged perpendicularly relative to the second axis, and
- a rotational bearing member (180) arranged between the gear wheel member and the ball housing and perpendicularly relative to the second axis,
wherein the first and second circumferential ball bearing surfaces in combination with corresponding bearing surfaces (145, 135) on the ball housing form first and second circumferential bearings which in combination provides the ball joint, and
wherein the friction of the rotational bearing is lower than the rotational friction of the second circumferential bearing.

4. A transmission as in claim 2, wherein the radius of the first circumferential bearing is smaller than the radius of the second circumferential bearing.

5. A drug delivery device comprising a transmission as in any of claims 1-4, further comprising:
- a compartment (19) adapted to receive a drug-filled cartridge (20), the drug-filled cartridge comprising a body portion, an axially displaceable piston (11), and a distal outlet portion adapted to be arranged in fluid communication with a flow conduit, and
- an electronically controlled drive arrangement (110) adapted to rotate the first gear wheel,
- wherein the rod is adapted to directly or indirectly engage and axially move the piston of a loaded drug-filled cartridge to thereby expel drug from the cartridge,

6. Drug delivery device as in claim 5, further comprising setting means allowing a user to set a dose of drug to be expelled.

7. Drug delivery device as in claim 5 or 6, wherein the compartment comprises a distal opening allowing a drug-filled cartridge to be received in a proximal direction.

## Patentansprüche

1. Getriebe (100) für eine motorisierte Arzneimittelabgabevorrichtung, wobei das Getriebe Folgendes umfasst:
- eine erste Achse und eine zweite Achse, die in einer Bezugsposition parallel zueinander angeordnet sind,
- ein erstes Zahnrad (160) mit:
- einer ersten Mittelebene, und
- einer der ersten Achse entsprechenden Drehachse,
- ein zweites Zahnrad (170) mit:
- einer zweiten Mittelebene, und
- einer der zweiten Achse entsprechenden Bezugsdrehachse,
**dadurch gekennzeichnet, dass** das zweite Zahnrad (170) eine der zweiten Achse entsprechende Gewindebohrung (171) aufweist, wobei das Getriebe ferner Folgendes umfasst:
- eine nicht drehend angeordnete Stange (120) mit einem Außengewinde (121) entlang mindestens einem Teil ihrer Länge, wobei die Stange in einer Bezugsposition entsprechend der zweiten Achse und in Gewindeeingriff mit der Gewindebohrung angeordnet ist, wodurch eine Drehung des zweiten Zahnrads eine axiale Bewegung der Stange bereitstellt,
wobei:
- das erste Zahnrad und das zweite Zahnrad in einer Bezugsposition so angeordnet sind, dass die erste Mittelebene und die zweite Mittelebene in einer gemeinsamen Ebene liegen und in Dreheingriff miteinander stehen,
- das zweite Zahnrad und die Stange in Kombination so angeordnet sind, dass sie entsprechend einem Mittelpunkt, der vom Kreuzungspunkt der zweiten Achse und der zweiten Mittelebene definiert wird, schwenken, wodurch die Stange, mit den Zahnrädern in Dreheingriff, so angeordnet werden kann, dass sie nicht mit der ersten Achse ausgerichtet ist.

2. Getriebe nach Anspruch 1, umfassend eine Kugelgelenkanordnung mit:
- einem Kugelabschnitt (170, 180, 190), der das zweite Zahnrad umfasst, und
- einem Kugelgehäuse (130, 140),
wobei das Kugelgelenk es dem zweiten Zahnrad und der Stange in Kombination gestattet, entsprechend dem Mittelpunkt zu schwenken.

3. Getriebe nach Anspruch 2, wobei der Kugelabschnitt Folgendes umfasst:
- ein Zahnradelement (170), umfassend die Gewindebohrung (171) und eine erste umlaufende Kugellagerfläche (175), die bezüglich der zweiten Achse senkrecht angeordnet ist,
- ein Lagergehäuse (190), das eine zweite umlaufende Kugellagerfläche (195) umfasst, die bezüglich der zweiten Achse senkrecht angeordnet ist, und
- ein Drehlagerelement (180), das zwischen dem Zahnradelement und dem Kugelgehäuse und bezüglich der zweiten Achse senkrecht angeordnet ist,
wobei die erste und die zweite umlaufende Kugellagerfläche in Kombination mit entsprechenden Lagerflächen (145, 135) auf dem Kugelgehäuse erste und zweite umlaufende Lager bilden, die in Kombination das Kugelgelenk bereitstellen, und
wobei die Reibung des Drehlagers geringer als die Drehreibung des zweiten umlaufenden Lagers ist.

4. Getriebe nach Anspruch 2, wobei der Radius des ersten umlaufenden Lagers kleiner als der Radius des zweiten umlaufenden Lagers ist.

5. Arzneimittelabgabevorrichtung, umfassend ein Getriebe nach einem der Ansprüche 1-4, ferner umfassend:
- eine Kammer (19), die zur Aufnahme einer mit Arzneimittel gefüllten Kartusche (20) ausgelegt ist, wobei die mit Arzneimittel gefüllte Kartusche einen Körperabschnitt, einen axial verschiebbaren Kolben (11) und einen distalen Auslassabschnitt umfasst, der zur Anordnung in Fluidverbindung mit einer Strömungsleitung ausgelegt ist, und
- eine elektronisch gesteuerte Antriebsanordnung (110), die zum Drehen des ersten Zahnrads ausgelegt ist,
- wobei die Stange dazu ausgelegt ist, den Kolben einer geladenen mit Arzneimittel gefüllten Kartusche direkt oder indirekt in Eingriff zu nehmen und axial zu bewegen, um dadurch Arzneimittel aus der Kartusche auszutreiben,

6. Arzneimittelabgabevorrichtung nach Anspruch 5, ferner umfassend Einstellmittel, mit denen ein Benutzer eine Dosis des auszustoßenden Arzneimittels einstellen kann.

7. Arzneimittelabgabevorrichtung nach Anspruch 5 oder 6, wobei die Kammer eine distale Öffnung umfasst, die die Aufnahme einer mit Arzneimittel gefüllten Kartusche in einer proximalen Richtung gestattet.

## Revendications

1. Transmission (100) pour un dispositif d'administration de médicament motorisé, la transmission comprenant :
- un premier axe et un second axe qui, dans une position de référence, sont agencés parallèlement l'un à l'autre,
- une première roue d'engrenage (160) ayant :
- un premier plan central, et
- un axe de rotation correspondant au premier axe,
- une seconde roue d'engrenage (170) ayant :
- un second plan central, et
- un axe de rotation de référence correspondant au second axe,
**caractérisée en ce que**
la seconde roue d'engrenage (170) possède un alésage fileté (171) correspondant au second axe, la transmission comprenant en outre :
- une tige agencée non rotative (120) ayant un filet externe (121) sur au moins une partie de sa longueur, la tige dans une position de référence étant agencée par rapport au second axe et en prise filetée avec l'alésage fileté, la rotation de la seconde roue d'engrenage permettant de ce fait un mouvement axial de la tige,
dans laquelle
- la première roue d'engrenage et la seconde roue d'engrenage dans une position de référence sont agencées avec le premier plan central et le second plan central dans un plan commun et en prise rotative l'une avec l'autre,
- la seconde roue d'engrenage et la tige combinées sont conçues pour pivoter par rapport à un point central défini par l'intersection du second axe et du second plan central,
moyennant quoi la tige, avec les roues d'engrenage en prise rotative, peut être agencée en dehors de l'alignement sur le premier axe.

2. Transmission selon la revendication 1, comprenant un ensemble joint à rotule comportant :
- une partie rotule (170, 180, 190) comprenant la seconde roue d'engrenage, et
- un logement (130, 140) de rotule,
moyennant quoi le joint à rotule permet que la seconde roue d'engrenage et la tige combinées pivotent par rapport au point central.

3. Transmission selon la revendication 2, dans laquelle la partie rotule comprend :
- un élément de roue d'engrenage (170) comprenant l'alésage fileté (171) et une première surface de palier à billes circonférentiel (175) agencée perpendiculairement au second axe,
- un logement (190) de palier comprenant une seconde surface de palier à billes circonférentiel (195) agencée perpendiculairement au second axe, et
- un élément de palier rotatif (180) agencé entre l'élément de roue d'engrenage et le logement de rotule et perpendiculairement au second axe,
dans laquelle les première et seconde surfaces de palier à billes circonférentiel associées aux surfaces de palier (145, 135) correspondantes sur le logement de rotule forment des premier et second paliers circonférentiels qui, combinés, forment le joint à rotule, et
dans laquelle la friction du palier rotatif est inférieure à la friction de rotation du second palier circonférentiel.

4. Transmissions selon la revendication 2, dans laquelle le rayon du premier palier circonférentiel est plus petit que le rayon du second palier circonférentiel.

5. Dispositif d'administration de médicament comprenant une transmission selon l'une quelconque des revendications 1 à 4, comprenant en outre :
- un compartiment (19) conçu pour accueillir une cartouche (20) remplie de médicament, la cartouche remplie de médicament comprenant une partie corps, un piston pouvant se déplacer dans le sens axial (11) et une partie orifice de sortie distale conçue pour être agencée en communication fluidique avec un conduit d'écoulement,
et
- un agencement d'entraînement à commande électronique (110) conçu pour faire tourner la première roue d'engrenage,
- dans lequel la tige est conçue pour entrer en prise de manière directe ou indirecte avec le piston d'une cartouche remplie de médicament chargée et pour déplacer celui-ci de façon axiale afin de ce fait d'expulser le médicament de la cartouche,

6. Dispositif d'administration de médicament selon la revendication 5, comprenant en outre des moyens de paramétrage permettant à un utilisateur de paramétrer une dose de médicament à expulser.

7. Dispositif d'administration de médicament selon la revendication 5 ou 6, dans lequel le compartiment comprend une ouverture distale permettant qu'une cartouche remplie de médicament soit accueillie dans une direction proximale.
